# FASCICULE DE BREVET EUROPEEN

(11) **EP 1 066 814 B1**
(45) Date de publication et mention de la délivrance du brevet: **08.09.2004**
(21) Numéro de dépôt: 00401594.7
(22) Date de dépôt: 06.06.2000
(51) Int. Cl.: A61K 7/00, A61K 7/032, A61K 7/48

(54) **Mascara comprenant un polyuréthane et des fibres**
Wimperntusche enthaltend ein Polyurethan und Fibern
Mascara containing a polyurethane and fibres

(30) Priorité: 08.07.1999 FR 9908959
(43) Date de publication de la demande: 10.01.2001
(73) Titulaire: L'OREAL, 75008 Paris (FR)
(72) Inventeur: Collin, Nathalie, 92330 Sceaux (FR)
(74) Mandataire: Kromer, Christophe

(56) Documents cités:
- WO-A-97/29734
- US-A- 4 659 562
- US-A- 5 874 072
- PATENT ABSTRACTS OF JAPAN vol. 012, no. 110 (C-486), 8 avril 1988 (1988-04-08) & JP 62 238211 A (KOBAYASHI KOOC:KK), 19 octobre 1987 (1987-10-19)

## Description

La présente invention concerne une composition de maquillage ou de soin des matières kératiniques comprenant une dispersion aqueuse de polyuréthane et des fibres. L'invention se rapporte également à l'utilisation de cette composition pour le maquillage des matières kératiniques, notamment les phanères, ainsi qu'à un procédé de maquillage ou de soin cosmétique de ces dernières. La composition et le procédé de maquillage selon l'invention sont plus particulièrement destinés aux fibres kératiniques, notamment sensiblement longitudinales, d'êtres humains telles que les cils, les sourcils et les cheveux, y compris les faux-cils et les postiches. La composition peut être une composition cosmétique de maquillage ou bien encore une composition cosmétique de soin des matières kératiniques. Plus spécialement, l'invention porte sur un mascara.

Il est connu du document JP-A-3-153613 d'utiliser des fibres dans des compositions de mascara pour conférer un effet d'allongement et d'épaississement aux cils. Les documents JP-A- 57-158714 et JP-9-263518 décrivent des compositions de mascara comprenant des fibres et des polymères en dispersion aqueuse de type acrylique. On connaît également du documents WO 97/29734 des compositions cosmétiques, notamment de mascara, comprenant des microfibrilles d'origibne naturelle et un polymère filmogène acrylique en dispersion aqueuse. Toutefois, le maquillage obtenu avec ces mascaras n'est pas résistant à l'eau, lors de baignades ou de douches par exemple, et/ou aux larmes et/ou à la transpiration. Le maquillage ainsi fragilisé ne présente plus alors une bonne tenue dans le temps.

Le but de la présente invention est de proposer une composition de maquillage des fibres kératiniques, notamment des cils, conduisant à un maquillage présentant une bonne tenue dans le temps et résistant à l'eau, nottament sous frottement, tout en conférant un bon allongement des cils.

Les inventeurs ont découverts qu'une telle composition pouvait être obtenue en utilisant un polymère filmogène de polyuréthane associé à des fibres. La composition présente de bonnes propriétés cosmétiques, notamment adhère bien aux cils et les gaine sans former de paquets. Le maquillage ne s'effrite pas après une journée et est résistant à l'eau.

Plus précisément, l'invention a pour objet un procédé cosmétique de maquillage ou de soin des matières kératiniques comprenant l'application sur les matières kératiniques une composition comprenant, dans un milieu physiologiquement acceptable, un polymère filmogène sous forme de particules en dispersion aqueuse et des fibres, caractérisé par le fait que le polymère filmogène est un polyuréthane.

L'invention a aussi pour objet une composition cosmétique comprenant, dans un milieu physiologiquement acceptable, un polymère filmogène sous forme de particules en dispersion aqueuse, une cire et des fibres, caractérisée par le fait que le polymère filmogène est un polyuréthane.

L'invention a également pour objet l'utilisation d'un polymère filmogène de polyuréthane sous forme de particules en dispersion aqueuse et de fibres dans une composition cosmétique de maquillage ou de soin des matières kératiniques pour obtenir un film déposé sur les matières kératiniques résistant à l'eau, notamment sous frottement, et/ou aux larmes et/ou à la transpiration.

De façon surprenante, l'utilisation d'une dispersion de polyuréthane associée à des fibres permet de rendre résistant à l'eau une composition aqueuse. Or jusqu'à ce jour, les mascaras dits "waterproof" étaient des compositions anhydres.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres peuvent avoir une longueur allant de 0,1 mm à 10 mm, de préférence de 1 mm à 5 mm et mieux de 1 mm à 3,5 mm. Leur section peut être comprise dans un cercle de diamètre allant de 500 nm à 500 µm, de préférence allant de 10 nm à 100 µm et mieux de 20 µm à 50 µm. Le poids des fibres est souvent donné en denier ou décitex.

Les fibres peuvent être celles utilisées dans la fabrication des textiles et notamment des fibres de soie, de coton, de laine, de lin, des fibres de cellulose (ou rayonne) - notamment extraites notamment du bois, des légumes ou des algues -, de polyamide (Nylon ®), de viscose, d'acétate notamment d'acétate de rayonne, de poly-(p-phénylène-téréphtalamide) (ou d'aramide) notamment de Kevlar ®, de polymère acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène (comme le Téflon ®⁾, de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

Par ailleurs, les fibres peuvent être traités ou non en surface, enrobées ou non. Comme fibres enrobées utilisables dans l'invention, on peut citer des fibres de polyamides enrobées de sulfure de cuivre pour un effet anti-statique (par exemple le R-STAT de chez Rhodia) ou un autre polymère permettant une organisation particulière des fibres (traitement de surface spécifique) ou traitement de surface induisant des effets de couleurs/hologrammes (fibre Lurex de chez Sildorex, par exemple).

De préférence, on utilise des fibres d'origine synthétiques et en. particulier des fibres organiques, comme celles utilisées en chirurgie. Avantageusement, on peut utiliser des fibres insolubles dans l'eau.

Les fibres utilisables dans la composition selon l'invention sont préférentiellement des fibres de polyamide ou de cellulose. Leur longueur peut aller de 0.1 à 5 mm, de préférence de 0.25 à 1.6 mm et leur diamètre moyen peut aller de 5 à 50 µm. En particulier, on peut utiliser les fibres de polyamide commercialisées par les Etablissements P. Bonte sous le nom Polyamide 0.9 Dtex 0.3 mm, ayant un diamètre moyen de 6pm, un poids d'environ (0.9 dtex) et une longueur allant de 0,3mm à 1,5mm. On peut aussi utiliser les fibres de poly-p-phénylène téréphtamide de diamètre moyen de 12 µm et de longueur d'environ 1,5 mm comme celles vendues sous le nom de Kevlar Floc par la société Du Pont Fibres ou bien encore les fibres de celluloses (ou de rayonne) ayant un diamètre moyen de 50 µm et une longueur allant de 0,5 mm à 6 mm comme celles vendues sous le nom de Natural rayon flock fiber RC1BE - N003 - M04 par la société Claremont Flock. On peut également utiliser des fibres de polyéthylène comme celles vendues sous le nom de Shurt Stuff 13 099 F par la société Mini Fibers.

Les fibres peuvent êtres présentes dans la compositon selon l'invention en une teneur allant de 0,1 à 10 % en poids, par rapport au poids total de la composition, de préférence de 0,3 % à 5 % en poids.

Dans la présente demande, on entend par "polymère filmogène", un polymère apte à former à lui seul ou en présence d'un agent auxiliaire de filmification, un film isolable.

Par polymère sous forme de particules en dispersion aqueuse, connu généralement sous le nom de latex ou pseudolatex, on entend une phase contenant de l'eau et éventuellement un composé soluble dans l'eau, dans laquelle est dispersé directement le polymère sous forme de particules.

Le polyuréthane utilisé selon l'invention peut être avantageusement choisi parmi les polyester-polyuréthanes et les polyéther-polyuréthanes. Le polyuéthane peut être de préférence un polyuréthane anionique. En particulier, le polyuréthane peut être choisi parmi les polyuréthanes aptes à former un film ayant une dureté allant de 10 secondes à 200 secondes.

La dureté du film de polymère est mesurée sur un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polymère radicalaire. La dureté du film est mesurée selon la norme ASTM D-43-66, ou la norme NF-T 30-016 (octobre 1981), à l'aide d'un pendule de Persoz.

Selon un premier mode de réalisation de l'invention, le polyuréthane peut avoir une reprise en eau inférieure ou égale à 30 %, et notamment allant de 0,5 % à 15 %. Les polyester-polyuréthanes selon l'invention peuvent présenter de telles propriétés de reprise en eau. De tels polyuréthanes permettent d'obtenir un produit de maquillage présentant une bonne tenue dans le temps et une bonne résistance à l'eau. Avantageusement, on peut utiliser des polyester-polyuréthanes aptes à former un film ayant une dureté allant de 40 à 200 secondes, et mieux de 50 à 170 secondes.

Selon la présente demande, on entend par "reprise en eau du polyuréthane", le pourcentage d'eau absorbé par le polyuréthane après 10 minutes d'immersion dans l'eau, à 30 °C. La reprise en eau est mesurée pour une couche de 300 µm d'épaisseur (avant séchage) déposée sur une plaque puis séchée pendant 24 heures à 30 °C et à 50 % d'humidité relative ; des morceaux d'environ 1 cm2 découpés dans le film sec sont pesés (mesure de la masse M1) puis immergés dans l'eau pendant 10 minutes ; après immersion, le morceau de film est essuyé pour éliminer l'excédent d'eau en surface puis pesé (mesure de la masse M2). La différence M2 - M1 correspond à la quantité d'eau absorbée par le polymère. La reprise en eau est égale à [ (M2 - M1) / M1 ] x 100 et est exprimée en pourcentage de poids d'eau par rapport au poids de polymère.

Selon un deuxième mode de réalisation de l'invention, le polyuréthane peut avoir une reprise en eau supérieure à 30 %, de préférence de 30 % à 150 %, et mieux de 40 % à 100 %. Les polyéther-polyuréthanes selon l'invention peuvent présenter de telles propriétés de reprise en eau. De tels polyuréthanes permettent d'obtenir un produit de maquillage bien adhérent aux cils, présentant une bonne tenue dans le temps. Avantageusement, on peut utiliser des polyéther-polyuréthanes aptes à former un film ayant une dureté allant de 10 à 40 secondes, et mieux de 20 à 35 secondes.

Les particules de polyuréthane dispersées dans le milieu aqueux de la composition ont généralement une taille pouvant aller de 10 nm à 300 nm, et mieux de 20 nm à 200 nm.

Comme polyester-polyuréthane, on peut utiliser ceux vendus sous les dénominations "AVALURE UR-425", "AVALURE UR-430", "AVALURE UR-405", "AVALURE UR-410" par la société GOODRICH.

Comme polyéther-polyuréthane, on peut utiliser ceux vendus sous les dénominations "SANCURE 878", "AVALURE UR-450", "SANCURE 861" par la société GOODRICH.

Le polyuréthane peut être présent dans la composition du procédé selon l'invention en une teneur, en poids de matières sèches, allant de 1 % à 60 % en poids, par rapport au poids total de la composition, de préférence de 2 % à 25 % en poids, et mieux de 2 % à 10 % en poids.

La composition selon l'invention peut comprendre, en outre, au moins une cire. La cire peut être choisie parmi les cires d'origine animale, les cires d'origine végétale, les cires d'origine minérale, les cires synthétiques et les fractions diverses de cires d'origine naturelle. Les cires peuvent être présentes en une teneur allant de 0,5 % à 40 % en poids (notamment de 2 % à 40 % en poids), par rapport au poids total de la composition, de préférence de 5 % à 30 % en poids, et mieux de 10 % à 25 % en poids.

Avantageusement, la cire peut être choisie parmi les cires (I) ayant un point de fusion allant de 70 °C à 110 °C. Ces cires ont notamment une pénétrabilité à l'aiguille allant de 1 à 7, 5. La pénétrabilité à l'aiguille des cires est déterminée selon la norme française NF T 60-123 ou la norme américaine ASTM D 1321, à la température de 25 °C. Selon ces normes, la pénétrabilité à l'aiguille est la mesure de la profondeur, exprimée en dixièmes de millimètre, à laquelle une aiguille normalisée, pesant 2,5 g disposée dans un équipage mobile pesant 97,5 g et placée sur la cire à tester, pendant 5 secondes, pénètre dans la cire.

Les cires (I) peuvent par exemples être choisies notamment parmi la cire de son de riz, la cire de Carnauba, la cire d'Ouricuri, la cire de Candellila, les cires de Monatan, la cire de canne à sucre, certaines cires de polyéthylène qui répondent aux critères des cires (I).

Avantageusement, la composition selon l'invention peut comprendre une quantité de cires (I) allant de 0,1 % à 20 % en poids, par rapport au poids total de la composition, de préférence de 1 % à 10 % en poids.

Selon un mode de réalisation de la composition selon l'invention, la composition peut comprendre au moins une cire (la) ayant un point de fusion supérieur ou égal à 70 °C et inférieur à 83 °C et/ou une une cire (Ib) ayant un point de fusion allant de 83°C à 110°C.

Comme cire (la), on peut par exemple citer la cire de son de riz ou la cire de Candelilla. Comme cire (Ib), on peut citer par exemple la cire de Carnauba, la cire d'Ouricuri, les cires de Montan. On utilise de préférence la cire de Carnauba.

Avantageusement, la composition selon l'invention peut comprendre un mélange de cires (I) contenant au moins une première cire (la) et au moins une deuxième cire (Ib) telles que définies précédemment.

Ledit mélange de cires (I) peut comprendre de 5 % à 50 % en poids de cire (la), par rapport au poids total dudit mélange de cires (I), et de 50 % à 95 % en poids de cire (Ib).

La composition peut comprendre, en outre, au moins une cire (II), dite cire molle, ayant un point de fusion supérieur ou égal à 45 °C et inférieur à 70 °C. La cire (II) peut avantageusement avoir une pénétrabilité à l'aiguille supérieure à 7,5, et de préférence inférieure ou égale à 217, mesurée selon les conditions définies précédemment pour les cires (I). Cette cire (II) permet notamment d'assouplir le revêtement déposé sur les cils.

Ces cires (II) peuvent être notamment choisies parmi la cire d'abeilles, les cires de lanoline, les cires de paraffine, les cires de cérasine, les cires microcristallines, les ozokérites, les spermaceti, certaines cires de polyéthylène de poids moléculaire tel qu'elles répondent aux critères des cires II, les huiles végétales hydrogénées.

Parmi les huiles végétales hydrogénées, on peut citer les cires de jojoba hydrogénées et les huiles hydrogénées qui sont obtenues par hydrogénation catalytique de corps gras composés de chaîne grasse linéaire ou non en C₈-C₃₂ et qui ont les qualités correspondant à la définition des cires. On peut citer notamment l'huile de tournesol hydrogénée, l'huile de ricin hydrogénée, l'huile de coton hydrogénée, l'huile de coprah hydrogénée et la lanoline hydrogénée.

Avantageusement, la cire (I) et la cire (II) peuvent être présentes dans la composition selon un rapport pondéral cire (I) / cire (II) pouvant aller de 0,2 à 1, et de préférence de 0,4 à 0,7.

La composition peut contenir, en outre, au moins un polymère filmogène auxiliaire différent du polymère de polyuréthane défini précédemment, en une teneur pouvant aller de 0 % à 15 % en poids (notamment 0,1 % à 15 % en poids), par rapport au poids total de la composition, et de préférence de 0,1 % à 10 % en poids.

Comme polymère filmogène auxiliaire, on peut par exemple citer les
- les polymères de cellulose tels que l'hydroxyéthylcellulose, l'hydroxypropylcellulose, l'hydroxypropyl éthylcellulose, l'éthylhydroxyéthyl-cellulose ;
- les polymères ou copolymères d'esters acryliques, tels que les polyacrylates ou les polyméthacrylates ;
- les polymères vinyliques, comme les polyvinylpyrrolidones, les copolymères de vinylpyrrolidone et d'acétate de vinyle ; l'alcool polyvinylique ;
- les polyesters, les polyamides, et les résines époxyesters ;
- les polymères d'origine naturelle, éventuellement modifiés, tels que les gommes arabiques, la gomme de guar, les dérivés du xanthane, la gomme de karaya ;
- et leurs mélanges.

La composition du procédé selon l'invention peut se présenter sous la forme de dispersion cire-dans-eau, eau-dans cire, huile-dans-eau et eau-dans-huile. La teneur en eau dans la composition peut aller de 1 à 95 % en poids, par rapport au poids total de la composition, et mieux de 10 à 80 % en poids.

La composition selon l'invention peut comprendre en outre au moins une huile volatile. On entend par "huile volatile" une huile susceptible de s'évaporer à température ambiante d'un support sur lequel elle a été appliquée, autrement dit une huile ayant une tension de vapeur mesurable à température ambiante.

On peut notamment utiliser une ou plusieurs huiles volatiles à température ambiante et pression atmosphérique ayant par exemple une tension de vapeur, à pression et température ambiante > 0 mm de Hg (0 Pa) et en particulier allant de 10⁻³ à 300 mm de Hg (0,13 Pa à 40.000 Pa), à condition que la température d'ébullition soit supérieure à 30°C. Ces huiles volatiles sont favorables à l'obtention d'un film à propriétés "sans transfert" total et de bonne tenue. Ces huiles volatiles facilitent, en outre, l'application de la composition sur la peau, les muqueuses, les phanères. Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles préférées convenant pour la composition selon l'invention sont en particulier les huiles hydrocarbonées ayant de 8 à 16 atomes de carbones, et notamment les isoalcanes en C₈-C₁₆ (ou isoparaffines) et les esters ramifiés en C₈-C₁₆ comme l'isododécane (encore appelé 2,2,4,4,6-pentaméthylheptane), l'isodécane, l'isohexadécane, le néo pentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées.

Comme huiles volatiles, on peut aussi utiliser les silicones volatiles, comme par exemple les huiles de silicones cycliques et volatiles, notamment celles ayant une viscosité ≤ 8 centistokes (8 10⁻⁶ m²/s), telles que l'octaméthylcyclotétrasiloxane, le décaméthylcyclopentasiloxane, l'hexadécaméthylcyclohexasiloxane, les silicones linéaires volatiles telles que l'octaméthyltrisiloxane, l'heptaméthylhexyltrisiloxane, l'heptaméthyloctyltrisiloxane, le décaméthyltétrasiloxane, ou bien encore les huiles volatiles fluorées telles que le nonafluorométhoxybutane ou le perfluorométhylcyclopentane.

L'huile volatile peut être présente dans la composition selon l'invention en une teneur allant de 0 % à 80 % en poids (notamment de 1 % à 80 %), par rapport au poids total de la composition, de préférence de 0 % à 65 % en poids (notamment de 1 % à 65 %).

La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :
- les huiles hydrocarbonées d'origine animale telle que le perhydrosqualène ;
- les huiles hydrocarbonées d'origine végétale telles que les triglycérides liquides d'acides gras de 4 à 10 atomes de carbone comme les triglycérides des acides heptanoïques ou octanoïque, ou encore les huiles de tournesol, de pépins de raisin, de sésame, de maïs, d'abricot, de ricin, d'avocat, d'olive ou de germes de céréales de soja, l'huile d'amande douce, de palme, de colza, de coton, de noisette, de macadamia, de jojoba, les triglycérides des acides caprylique/caprique comme ceux vendus par la société Stéarineries Dubois ou ceux vendus sous les dénominations Miglyol 810, 812 et 818 par la société Dynamit Nobel, l'huile de beurre de karité ;
- les hydrocarbures linéaires ou ramifiés, d'origine minérale ou synthétique tels que la vaseline, les polydécènes, le polyisobutène hydrogéné tel que le parléam ;
- les esters et les éthers de synthèse comme les huiles de formule R₁COOR₂ dans laquelle R₁ représente le reste d'un acide gras supérieur comportant de 6 à 29 atomes de carbone et R2 représente une chaîne hydrocarbonée contenant de 3 à 30 atomes de carbone, telles que l'huile de Purcellin, le myristate d'isopropyle, le palmitate d'isopropyle, le stéarate de butyle, le laurate d'hexyle, l'adipate de diisopropyle, l'isononate d'isononyle, le palmitate de 2-éthyl-hexyle, le laurate de 2-hexyl-décyle, le palmitate de 2-octyl-décyle, le myristate ou le lactate de 2-octyldodécyle ; les esters de polyols comme le dioctanoate de propylène glycol, le diheptanoate de néopentylglycol, le diisonanoate de diéthylène glycol et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide myristique, l'acide palmitique, l'acide stéarique, l'acide béhénique, l'acide oléique, l'acide linoléique, l'acide linolénique ou l'acide isostéarique ; et leurs mélanges.

Les huiles de silicone non volatiles utilisables dans la composition selon l'invention peuvent être des huiles de faible viscosité telles que les polysiloxanes linéaires dont le degré de polymérisation est de préférence de 6 à 2000 environ. On peut citer, par exemple, les polydiméthylsiloxanes (PDMS) de viscosité supérieure à 10 mPa.s, les phényl diméthicones, les phényl triméthicones, les polyphénylméthylsiloxanes et leurs mélanges.

Les huiles fluorées utilisables dans l'invention sont notamment des huiles fluorosiliconées, des polyéthers fluorés, des silicones fluorées telles que décrit dans le document EP-A-847752.

Les huiles non volatiles peuvent être présentes dans la composition selon l'invention en une teneur allant de 0 % à 50 % en poids (notamment 0,1 à 50 %), par rapport au poids total de la composition, de préférence de 0 % à 20 % en poids (notamment 0,1 % à 20 %).

La composition selon l'invention peut contenir des agents tensioactifs émulsionnants présents notamment en une proportion allant de 2 à 30 % en poids par rapport au poids total de la composition, et mieux de 5 % à 15 %. Ces agents tensioactifs peuvent être choisis parmi des agents tensioactifs anioniques ou non ioniques. On peut se reporter au document « Encyclopedia of Chemical Technology, KIRK-OTHMER », volume 22, p.333-432, 3ème édition, 1979, WILEY, pour la définition des propriétés et des fonctions (émulsionnant) des tensioactifs, en particulier p.347-377 de cette référence, pour les tensioactifs anioniques et non-ioniques.

Les tensioactifs utilisés préférentiellement dans la composition selon l'invention sont choisis :
- parmi les tensioactifs non-ioniques : les acides gras, les alcools gras, les alcools gras polyéthoxylés ou polyglycérolés tels que des alcools stéarylique ou cétylstéarylique polyéthoxylés, les esters d'acide gras et de saccharose, les esters d'alkyl glucose, en particulier les esters gras de C₁-C₆ alkyl glucose polyoxyéthylénés, et leurs mélanges.
- parmi les tensioactifs anioniques : les acides gras en C₁₆-C₃₀ neutralisés par les amines, l'ammoniaque ou les sels alcalins, et leurs mélanges.

On utilise de préférence des tensioactifs permettant l'obtention d'émulsion huile-dans-eau ou cire-dans-eau.

La composition peut également comprendre au moins une matière colorante comme les composés pulvérulents, par exemple à raison de 0,01 à 25 % du poids total de la composition. Les composés pulvérulents peuvent être choisis parmi les pigments et/ou les nacres et/ou les charges habituellement utilisés dans les mascaras.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être choisies parmi celles bien connues de l'homme du métier et couramment utilisées dans les compositions cosmétiques. Comme charge, on peut notamment utiliser :
- le talc qui est un silicate de magnésium hydraté utilisé sous forme de particules généralement inférieures à 40 microns,
- les micas qui sont des aluminosilicates de compositions variées se présentant sous la forme d'écailles ayant des dimensions de 2 à 200 microns, de préférence de 5 à 70 microns et une épaisseur comprise entre 0,1 à 5 microns, de préférence de 0,2 à 3 microns, ces micas pouvant être d'origine naturelle telle que la muscovite la margarite, la roscoelithe, la lipidolithe, la biotite ou d'origine synthétique,
- l'amidon en particulier l'amidon de riz,
- le kaolin qui est un silicate d'aluminium hydraté qui se présente sous la forme de particules de forme isotrope ayant des dimensions généralement inférieures à 30 microns,
- les oxydes de zinc et de titane généralement utilisés sous la forme de particules ayant des dimensions ne dépassant pas quelques microns,
- le carbonate de calcium, le carbonate ou l'hydrocarbonate de magnésium,
- la cellulose microcristalline,
- la silice,
- les poudres de polymères synthétiques tels que le polyéthylène, les polyesters (l'isophtalate ou le téréphtalate de polyéthylène), les polyamides tels que ceux vendus sous la dénomination commerciale de "Nylon" ou de "Téflon" et les poudres de silicone.

La composition selon l'invention peut également contenir des ingrédients couramment utilisés en cosmétique, tels que les oligo-éléments, les adoucissants, les séquestrants, les parfums, les huiles, les silicones, les épaississants, les vitamines, les protéines, les céramides, les plastifiants, les agents de cohésion ainsi que les agents alcalinisants ou acidifiants habituellement utilisés dans le domaine cosmétique, les émollients, les conservateurs.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée selon les méthodes usuelles des domaines considérés.

L'invention est illustrée plus en détail dans les exemples suivants.

### Exemples 1 et 1' comparatifs :

On a préparé un mascara (exemple 1) selon l'invention et un mascara ne faisant pas partie de l'invention (exemple 1') ayant la composition suivante :

| | |
|---|---|
| - Cire de carnauba | 2,4 g |
| - Cire d'abeille | 3 g |
| - Cire de paraffine | 9, 5 g |
| - Amino-2 méthyl-2 propanediol-1,3 | 0,8 g |
| - Triéthanolamine | 2,4 g |
| - Acide stéarique | 6,6 g |
| - Polymères non-ioniques hydrosolubles | 1,4 g |
| - Polymère filmogène en dispersion aqueuse* | 5 g MA |
| - Fibres de polyamide (0,3 mm de long et 0,9 Dtex de la société Paul Bonte) | 1 g |
| - Mélange de diméthiconol dans du cyclopentasiloxane (15/85) (DC 1501 Fluid de la société DOW CORNING) | 8 g |
| - Pigments noirs | 7 g |
| - Conservateurs qs | |
| - Eau qsp | 100 g |

| | |
|---|---|
| * exemple 1 : polyester-polyuréthane vendu sous le nom AVALURE UR 405 par la société GOODRICH exemple-1' : copolymère acrylate d'éthyle/méthacrylate de méthyle vendu sous le nom de DAITOSOL 5000 AD par la société SAITO | |

On a appliqué chaque composition sur une éprouvette de cheveux puis après séchage, on a frotté chaque éprouvette avec un coton imbibé d'eau en effectuant 10 passages. On constate que le coton ayant frotté le mascara de l'exemple 1' est bien plus noir que celui ayant frotté le mascara de l'exemple 1. Ce dernier est donc plus résistant à l'eau, même sous frottement, que le mascara de l'exemple 1'.

### Exemple 2 :

On a préparé un mascara ayant la composition suivante :
- Cire de carnauba 2,4 g
- cire d'abeille 3 g
- Cire de paraffine 9,5 g
- Amino-2 méthyl-2 propanediol-1,3 0,8 g
- Triéthanolamine 2,4 g
- Acide stéarique 6,6 g
- Polymères non-ioniques hydrosolubles 1,4 g
- polyester-polyuréthane en dispersion aqueuse AVALURE UR 405 de GOODRICH 5 g MA
- Fibres de cellulose (Natural rayon flock fiber RC1BE - N003
   - M04 par la société Claremont Flock) 1 g
- Pigments 6 g
- Conservateurs qs
- Eau qsp 100 g

Ce mascara permet d'obtenir un maquillage bien résistant aux larmes et à la sueur et de bonne tenue : il confère, en outre, aux cils un bon allongement .

### Exemple 3 :

On a préparé un mascara ayant la composition suivante :
- dispersion aqueuse de polyester-polyuréthane à 49 % de matières sèches (AVALURE UR-425 de GOODRICH) 35,8 g MA
- Fibres de cellulose (Natural rayon flock fiber RC1BE - N003
   - M04 par la société Claremont Flock) 1 g
- Hydroxyéthyl cellulose (Cellosize QP 4400 H d'AMERCHOL) 1,82 g
- Silice pyrogénée (AEROSIL 200 de DEGUSSA) 1,82 g
- éthanol 5 g
- propylène glycol 4,05 g
- acide citrique 0,15 g
- pigments 4 g
- conservateurs qs
- eau qsp 100 g

Ce mascara permet d'obtenir un maquillage de bonne tenue pendant au moins un jour et les cils présentent un bon allongement.

## Revendications

1. Procédé cosmétique de maquillage ou de soin des matières kératiniques comprenant l'application sur les matières kératiniques d'une composition comprenant, dans un milieu physiologiquement acceptable, un polymère filmogène sous forme de particules en dispersion aqueuse et des fibres, **caractérisé par le fait que** le polymère filmogène est un polyuréthane.

2. Procédé selon la revendication 1, **caractérisé par le fait que** le polyuréthane est choisi dans le groupe formé par les polyester-polyuréthanes et les polyéther-polyuréthanes.

3. Procédé selon l'une des revendications 1 ou 2 **caractérisé par le fait que** le polyuréthane est un polyuréthane anionique.

4. Procédé selon l'une quelconque des revendications précédentes, **caractérisé par le fait que** le polyuréthane est apte à former un film ayant une dureté allant de 10 secondes à 200 secondes.

5. Procédé selon l'une quelconque des revendications précédentes, **caractérisé par le fait que** le polyuréthane est présent en une teneur, en poids de matières sèches, allant de 1 % à 60 % en poids, et mieux de 2 % à 25 % en poids, par rapport au poids total de la composition.

6. Procédé selon l'une quelconque des revendications précédentes, **caractérisé par le fait que** les fibres sont choisies parmi les fibres de soie, de coton, de laine, de lin, des fibres de cellulose, de polyamide, de viscose, d'acétate notamment d'acétate de rayonne, de poly-p-(phénylène-téréphtalamide), en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

7. Procédé selon l'une quelconque des revendications précédentes, **caractérisé par le fait que** les fibres sont des fibres d'origine synthétique.

8. Procédé selon l'une quelconque des revendications précédentes, **caractérisé par le fait que** les fibres sont des fibres de polyamide ou de cellulose.

9. Procédé selon l'une quelconque des revendications précédentes, **caractérisé par le fait que** les fibres ont une longueur allant de 0.1 à 10 mm, de préférence de 1 à 5 mm.

10. Procédé selon l'une quelconque des revendications précédentes, **caractérisé par le fait que** les fibres ont une section comprise dans un cercle de diamètre allant de 500 nm à 500 µm.

11. Procédé selon l'une quelconque des revendications précédentes, **caractérisé par le fait que** les fibres ont une section circulaire ou polygonale.

12. Procédé selon l'une quelconque des revendications précédentes, **caractérisé par le fait que** les fibres sont présentes en une teneur allant de 0,1 % à 10 % en poids, par rapport au poids total de la composition, et mieux de 0,3 % à 5 % en poids.

13. Procédé selon l'une quelconque des revendications précédentes, **caractérisé par le fait que** la composition contient, en outre, une cire.

14. Procédé selon la revendication 13, **caractérisé par le fait que** la cire est présente en une teneur allant de 0,5 % à 40 % en poids, par rapport au poids total de la composition, de préférence de 5 % à 30 % en poids, et mieux de 10 % à 25 % en poids.

15. Procédé selon l'une des revendications 13 ou 14, **caractérisé par le fait que** la cire comprend au moins une cire (I) ayant un point de fusion allant de 70 °C à 110°C.

16. Procédé selon la revendication 15, **caractérisé par le fait que** la cire (I) est présente en une teneur allant de 0,1 % à 20 % en poids, par rapport au poids total de la composition.

17. Procédé selon l'une quelconque des revendications précédentes 13 à 16, **caractérisé par le fait que** la cire comprend au moins une cire (II) ayant un point de fusion supérieur ou égal à 45 °C et inférieur à 70 °C.

18. Procédé selon la revendication 17, **caractérisé par le fait que** la cire (II) est présente selon un rapport pondéral cire (I)/cire (II) allant de 0,2 à 1.

19. Procédé selon l'une quelconque des revendications précédentes, **caractérisé par le fait que** la composition comprend, en outre, au moins un polymère filmogène auxiliaire différent du polymère de polyuréthane.

20. Procédé selon l'une quelconque des revendications précédentes, **caractérisé par le fait que** la composition comprend de l'eau en une teneur allant de 1 % à 95 % en poids, par rapport au poids total de la composition, et mieux de 10 à 80 % en poids.

21. Procédé selon l'une quelconque des revendications précédentes, **caractérisé par le fait que** la composition est sous forme d'une émulsion cire-dans-eau, eau-dans-cire, huile-dans-eau, eau-dans-huile.

22. Procédé selon l'une quelconque des revendications précédentes, **caractérisé par le fait que** la composition comprend, en outre, au moins une huile volatile.

23. Procédé selon l'une quelconque des revendications précédentes, **caractérisée par le fait que** la composition comprend, en outre, au moins un tensioactif émulsionnant.

24. Procédé selon la revendication 23, **caractérisée par le fait que** le tensioactif émulsionnant est présent en une teneur allant de 2 % à 30 % en poids, par rapport au poids total de la composition.

25. Procédé selon l'une quelconque des revendications précédentes, **caractérisée par le fait que** la composition comprend au moins un additif choisi dans le groupe formé par les vitamines, les oligo-éléments, les adoucissants, les séquestrants, les parfums, les huiles, les vitamines, les épaississants, les protéines, les céramides, les plastifiants, les agents de cohésion, les agents alcalinisants ou acidifiants, les charges, les pigments, les émollients, les conservateurs, et leurs mélanges.

26. Composition cosmétique comprenant, dans un milieu physiologiquement acceptable, un polymère filmogène sous forme de particules en dispersion aqueuse, une cire et des fibres, **caractérisée par le fait que** le polymère filmogène est un polyuréthane.

27. Composition selon la revendication 26, **caractérisée par le fait que** le polyuréthane est choisi dans le groupe formé par les polyester-polyuréthanes et les polyéther-polyuréthanes.

28. Composition selon la revendication 26 ou 27, **caractérisée par le fait que** le polyuréthane est un polyuréthane anionique.

29. Composition selon l'une quelconque des revendications 26 à 28, **caractérisée par le fait que** le polyuréthane est apte à former un film ayant une dureté allant de 10 secondes à 200 secondes.

30. Composition selon l'une quelconque des revendications 26 à 29, **caractérisée par le fait que** le polyuréthane est présent en une teneur, en poids de matières sèches, allant de 1 % à 60 % en poids, et mieux de 2 % à 25 % en poids, par rapport au poids total de la composition.

31. Composition selon l'une quelconque des revendications 26 à 30, **caractérisée par le fait que** les fibres sont choisies parmi les fibres de soie, de coton, de laine, de lin, des fibres de cellulose, de polyamide, de viscose, d'acétate notamment d'acétate de rayonne, de poly-p(phénylène-téréphtalamide), en acrylique notamment de polyméthacrylate de méthyle ou de poly 2-hydroxyéthyl méthacrylate, de polyoléfine et notamment de polyéthylène ou de polypropylène, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

32. Composition selon l'une quelconque des revendications 26 à 31, **caractérisée par le fait que** les fibres sont des fibres d'origine synthétique.

33. Composition selon l'une quelconque des revendications 26 à 32, **caractérisée par le fait que** les fibres sont des fibres de polyamide ou de cellulose.

34. Composition selon l'une quelconque des revendications 26 à 33, **caractérisée par le fait que** les fibres ont une longueur allant de 0.1 à 10 mm, de préférence de 1 à 5 mm.

35. Composition selon l'une quelconque des revendications 26 à 34, **caractérisée par le fait que** les fibres ont une section comprise dans un cercle de diamètre allant de 500 nm à 500 µm.

36. Composition selon l'une quelconque des revendications 26 à 35, **caractérisée par le fait que** les fibres ont une section circulaire ou polygonale.

37. Composition selon l'une quelconque des revendications 26 à 36, **caractérisée par le fait que** les fibres sont présentes en une teneur allant de 0,1 % à 10 % en poids, par rapport au poids total de la composition, et mieux de 0,3 % à 5 % en poids.

38. Composition selon l'une quelconque des revendications 26 à 37, **caractérisée par le fait que** la cire est présente en une teneur allant de 0,5 % à 40 % en poids, par rapport au poids total de la composition, de préférence de 5 % à 30 % en poids, et mieux de 10 % à 25 % en poids.

39. Composition selon l'une quelconque des revendications 26 à 38, **caractérisée par le fait que** la cire comprend au moins une cire (I) ayant un point de fusion allant de 70 °C à 110°C.

40. Composition selon la revendication 39, **caractérisée par le fait que** la cire (I) est présente en une teneur allant de 0,1 % à 20 % en poids, par rapport au poids total de la composition.

41. Composition selon l'une quelconque des revendications précédentes 26 à 40, **caractérisée par le fait que** la cire comprend au moins une cire (II) ayant un point de fusion supérieur ou égal à 45 °C et inférieur à 70 °C.

42. Composition selon la revendication 41, **caractérisée par le fait que** la cire (II) est présente selon un rapport pondéral cire (I)/cire (II) allant de 0,2 à 1.

43. Composition selon l'une quelconque des revendications 26 à 42, **caractérisée par le fait qu'**elle comprend, en outre, au moins un polymère filmogène auxiliaire différent du polymère de polyuréthane.

44. Composition selon l'une quelconque des revendications 26 à 43, **caractérisée par le fait qu'**elle comprend de l'eau en une teneur allant de 1 % à 95 % en poids, par rapport au poids total de la composition, et mieux de 10 à 80 % en poids.

45. Composition selon l'une quelconque des revendications 26 à 44, **caractérisé en ce qu'**elle est sous forme d'une émulsion cire-dans-eau, eau-dans-cire, huile-dans-eau, eau-dans-huile.

46. Composition selon l'une quelconque des revendications 26 à 45, **caractérisée par le fait qu'**elle comprend, en outre, au moins une huile volatile.

47. Composition selon l'une quelconque des revendications 26 à 46, **caractérisée par le fait qu'**elle comprend, en outre, au moins un tensioactif émulsionnant.

48. Composition selon la revendication 47, **caractérisée par le fait que** le tensioactif émulsionnant est présent en une teneur allant de 2 % à 30 % en poids, par rapport au poids total de la composition.

49. Composition selon l'une quelconque des revendications 26 à 48, **caractérisée par le fait qu'**elle comprend au moins un additif choisi dans le groupe formé par les vitamines, les oligo-éléments, les adoucissants, les séquestrants, les parfums, les huiles, les vitamines, les épaississants, les protéines, les céramides, les plastifiants, les agents de cohésion, les agents alcalinisants ou acidifiants, les charges, les pigments, les émollients, les conservateurs, et leurs mélanges.

50. Composition selon l'une quelconque des revendications 26 à 49, **caractérisée par le fait que** la composition est une composition de maquillage ou une composition de cosmétique de soin des matières kératiniques.

51. Utilisation d'un polymère filmogène de polyuréthane sous forme de particules en dispersion aqueuse et de fibres dans une composition cosmétique de maquillage ou de soin des matières kératiniques pour obtenir un film déposé sur les matières kératiniques résistant à l'eau, notamment sous frottement, et/ou aux larmes et/ou à la transpiration.

## Patentansprüche

1. Kosmetisches Verfahren zum Schminken oder zur Pflege von Keratinsubstanzen, das umfasst, auf die Keratinsubstanzen eine kosmetische Zusammensetzung aufzubringen, die in einem physiologisch akzeptablen Medium ein filmbildendes Polymer in Form von Partikeln in wässriger Dispersion und Fasern enthält, **dadurch gekennzeichnet, dass** das filmbildende Polymer ein Polyurethan ist.

2. Verfahren nach Anspruch 1, **dadurch gekennzeichnet, dass** das Polyurethan unter den Polyester-Polyurethanen und Polyether-Polyurethanen ausgewählt ist.

3. Verfahren nach Anspruch 1 oder 2, **dadurch gekennzeichnet, dass** das Polyurethan ein anionisches Polyurethan ist.

4. Verfahren nach einem der vorhergehenden Ansprüche, **dadurch gekennzeichnet, dass** das Polyurethan einen Film mit einer Härte von 10 bis 200 Sekunden bilden kann.

5. Verfahren nach einem der vorhergehenden Ansprüche, **dadurch gekennzeichnet, dass** das Polyurethan in einem Trockensubstanzgehalt von 1 bis 60 Gew.-%, bezogen auf das Gesamtgewicht der Zusammensetzung, und besser 2 bis 25 Gew.-% enthalten ist.

6. Verfahren nach einem der vorhergehenden Ansprüche, **dadurch gekennzeichnet, dass** die Fasern unter den Seidenfasern, Baumwollfasern, Wollfasern, Leinenfasern, Cellulosefasern, Polyamidfasern, Viskosefasern, Acetatfasern und insbesondere Fasern aus Reyonacetat, Fasern aus Poly(p-phenylen-terephthalamid), Acrylfasern, insbesondere Fasern aus Polymethylmethacrylat oder Poly-2-hydroxyethylmethacrylat, Polyolefinfasern und insbesondere Fasern aus Polyethylen oder Polypropylen, Siliciumdioxidfasern, Kohlenstofffasern und insbesondere aus Kohlenstoff in Form von Graphit, Polytetrafluorethylenfasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymergemischen ausgewählt sind.

7. Verfahren nach einem der vorhergehenden Ansprüche, **dadurch gekennzeichnet, dass** die Fasern synthetischer Herkunft sind.

8. Verfahren nach einem der vorhergehenden Ansprüche, **dadurch gekennzeichnet, dass** die Fasern Polyamidfasern oder Cellulosefasern sind.

9. Verfahren nach einem der vorhergehenden Ansprüche, **dadurch gekennzeichnet, dass** die Fasern eine Länge von 0,1 bis 10 mm und vorzugsweise 1 bis 5 mm aufweisen.

10. Verfahren nach einem der vorhergehenden Ansprüche, **dadurch gekennzeichnet, dass** die Fasern einen Querschnitt besitzen, der in einen Kreis mit einem mittleren Durchmesser von 500 nm bis 500 µm einbeschrieben werden kann.

11. Verfahren nach einem der vorhergehenden Ansprüche, **dadurch gekennzeichnet, dass** die Fasern einen kreisförmigen oder polygonalen Querschnitt besitzen.

12. Verfahren nach einem der vorhergehenden Ansprüche, **dadurch gekennzeichnet, dass** die Fasern in einer Menge von 0,1 bis 10 Gew.-%, bezogen auf das Gesamtgewicht der Zusammensetzung, und besser 0,3 bis 5 Gew.-%, enthalten sind.

13. Verfahren nach einem der vorhergehenden Ansprüche, **dadurch gekennzeichnet, dass** die Zusammensetzung ferner mindestens ein Wachs enthält.

14. Verfahren nach Anspruch 13, **dadurch gekennzeichnet, dass** das Wachs in einem Mengenanteil von 0, 5 bis 40 Gew.-%, bezogen auf das Gesamtgewicht der Zusammensetzung, vorzugsweise 5 bis 30 Gew.-% und besser 10 bis 25 Gew.-% enthalten ist.

15. Verfahren nach einem der Ansprüche 13 oder 14, **dadurch gekennzeichnet, dass** das Wachs mindestens ein Wachs (I) mit einem Schmelzpunkt von 70 bis 110 °C umfasst.

16. Verfahren nach Anspruch 15, **dadurch gekennzeichnet, dass** das Wachs (I) in einer Menge von 0,1 bis 20 Gew.-%, bezogen auf das Gesamtgewicht der Zusammensetzung, vorliegt.

17. Verfahren nach einem der Ansprüche 13 bis 16, **dadurch gekennzeichnet, dass** das Wachs mindestens ein Wachs (II) mit einem Schmelzpunkt von 45 °C oder darüber und unter 70 °C umfasst.

18. Verfahren nach Anspruch 17, **dadurch gekennzeichnet, dass** das Wachs (II) in einem Gewichtsverhältnis von Wachs (I)/Wachs (II) von 0,2 bis 1 vorliegt.

19. Verfahren nach einem der vorhergehenden Ansprüche, **dadurch gekennzeichnet, dass** die Zusammensetzung ferner mindestens ein zusätzliches filmbildendes Polymer enthält, das von dem Polyurethanpolymer verschieden ist.

20. Verfahren nach einem der vorhergehenden Ansprüche, **dadurch gekennzeichnet, dass** die Zusammensetzung Wasser in einer Menge von 1 bis 95 Gew.-%, bezogen auf das Gesamtgewicht der Zusammensetzung, und besser 10 bis 80 Gew.-% enthält.

21. Verfahren nach einem der vorhergehenden Ansprüche, **dadurch gekennzeichnet, dass** die Zusammensetzung als Wachs-in-Wasser-Emulsion, Wasser-in-Wachs- Emulsion, Öl-in Wasser-Emulsion und Wasser-in-Öl- Emulsion vorliegt.

22. Verfahren nach einem der vorhergehenden Ansprüche, **dadurch gekennzeichnet, dass** die Zusammensetzung ferner mindestens ein flüchtiges Öl enthält.

23. Verfahren nach einem der vorhergehenden Ansprüche, **dadurch gekennzeichnet, dass** die Zusammensetzung ferner mindestens einen emulgierenden grenzflächenaktiven Stoff enthält.

24. Verfahren nach Anspruch 23, **dadurch gekennzeichnet, dass** der emulgierende grenzflächenaktive Stoff in einer Menge von 2 bis 30 Gew.-%, bezogen auf das Gesamtgewicht der Zusammensetzung, enthalten ist.

25. Verfahren nach einem der vorhergehenden Ansprüche, **dadurch gekennzeichnet, dass** die Zusammensetzung ferner mindestens einen Zusatzstoff enthält, der unter den Vitaminen, Spurenelementen, beruhigenden Stoffen, Maskierungsmitteln, Parfums, Ölen, Vitaminen, Verdickungsmitteln, Proteinen, Ceramiden, Weichmachern, Kohäsionsmitteln, Alkalisierungs- und Ansäuerungsmitteln, Füllstoffen, Pigmenten, Emollientien, Konservierungsmitteln oder deren Gemischen ausgewählt ist.

26. Kosmetische Zusammensetzung, die in einem physiologisch akzeptablen Medium ein filmbildendes Polymer in Form von Partikeln in wässriger Dispersion, ein Wachs und Fasern enthält, **dadurch gekennzeichnet, dass** das filmbildende Polymer ein Polyurethan ist.

27. Zusammensetzung nach Anspruch 26, **dadurch gekennzeichnet, dass** das Polyurethan unter den Polyester-Polyurethanen und Polyether-Polyurethanen ausgewählt ist.

28. Zusammensetzung nach Anspruch 26 oder 27, **dadurch gekennzeichnet, dass** das Polyurethan ein anionisches Polyurethan ist.

29. Zusammensetzung nach einem der Ansprüche 26 bis 28, **dadurch gekennzeichnet, dass** das Polyurethan einen Film mit einer Härte von 10 bis 200 Sekunden bilden kann.

30. Zusammensetzung nach einem der Ansprüche 26 bis 29, **dadurch gekennzeichnet, dass** das Polyurethan in einem Trockensubstanzgehalt von 1 bis 60 Gew.-%, bezogen auf das Gesamtgewicht der Zusammensetzung, und besser 2 bis 25 Gew.-% enthalten ist.

31. Zusammensetzung nach einem der Ansprüche 26 bis 30, **dadurch gekennzeichnet, dass** die Fasern unter den Seidenfasern, Baumwollfasern, Wollfasern, Leinenfasern, Cellulosefasern, Polyamidfasern, Viskosefasern, Acetatfasern und insbesondere Fasern aus Reyonacetat, Fasern aus Poly(p-phenylen-terephthalamid), Acrylfasern, insbesondere Fasern aus Polymethylmethacrylat oder Poly-2-hydroxyethylmethacrylat, Polyolefinfasern und insbesondere Fasern aus Polyethylen oder Polypropylen, Siliciumdioxidfasern, Kohlenstofffasern und insbesondere aus Kohlenstoff in Form von Graphit, Polytetrafluorethylenfasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymergemischen ausgewählt sind.

32. Zusammensetzung nach einem der Ansprüche 26 bis 31, **dadurch gekennzeichnet, dass** die Fasern synthetischer Herkunft sind.

33. Zusammensetzung nach einem der Ansprüche 26 bis 32, **dadurch gekennzeichnet, dass** die Fasern Polyamidfasern oder Cellulosefasern sind.

34. Zusammensetzung nach einem der Ansprüche 26 bis 33, **dadurch gekennzeichnet, dass** die Fasern eine Länge von 0,1 bis 10 mm und vorzugsweise 1 bis 5 mm aufweisen.

35. Zusammensetzung nach einem der Ansprüche 26 bis 34, **dadurch gekennzeichnet, dass** die Fasern einen Querschnitt besitzen, der in einen Kreis mit einem mittleren Durchmesser von 500 nm bis 500 µm einbeschrieben werden kann.

36. Zusammensetzung nach einem der Ansprüche 26 bis 35, **dadurch gekennzeichnet, dass** die Fasern einen kreisförmigen oder polygonalen Querschnitt besitzen.

37. Zusammensetzung nach einem der Ansprüche 26 bis 36, **dadurch gekennzeichnet, dass** die Fasern in einer Menge von 0,1 bis 10 Gew.-%, bezogen auf das Gesamtgewicht der Zusammensetzung, und besser 0,3 bis 5 Gew.-%, enthalten sind.

38. Zusammensetzung nach einem der Ansprüche 26 bis 37, **dadurch gekennzeichnet, dass** das Wachs in einem Mengenanteil von 0,5 bis 40 Gew.-%, bezogen auf das Gesamtgewicht der Zusammensetzung, vorzugsweise 5 bis 30 Gew.-% und besser 10 bis 25 Gew.-% enthalten ist.

39. Zusammensetzung nach einem der Ansprüche 26 bis 38, **dadurch gekennzeichnet, dass** das Wachs mindestens ein Wachs (I) mit einem Schmelzpunkt von 70 bis 110 °C umfasst.

40. Zusammensetzung nach Anspruch 39, **dadurch gekennzeichnet, dass** das Wachs (I) in einer Menge von 0,1 bis 20 Gew.-%, bezogen auf das Gesamtgewicht der Zusammensetzung, vorliegt.

41. Zusammensetzung nach einem der Ansprüche 26 bis 40, **dadurch gekennzeichnet, dass** das Wachs mindestens ein Wachs (II) mit einem Schmelzpunkt von 45 °C oder darüber und unter 70 °C umfasst.

42. Zusammensetzung nach Anspruch 41, **dadurch gekennzeichnet, dass** das Wachs (II) in einem Gewichtsverhältnis von Wachs (I)/Wachs (II) von 0,2 bis 1 vorliegt.

43. Zusammensetzung nach einem der Ansprüche 26 bis 42, **dadurch gekennzeichnet, dass** die Zusammensetzung ferner mindestens ein zusätzliches filmbildendes Polymer enthält, das von dem Polyurethanpolymer verschieden ist.

44. Verfahren nach einem der nach einem der Ansprüche 26 bis 43, **dadurch gekennzeichnet, dass** die Zusammensetzung Wasser in einer Menge von 1 bis 95 Gew.-%, bezogen auf das Gesamtgewicht der Zusammensetzung, und besser 10 bis 80 Gew.-% enthält.

45. Verfahren nach einem der nach einem der Ansprüche 26 bis 44, **dadurch gekennzeichnet, dass** die Zusammensetzung als Wachsin-Wasser-Emulsion, Wasser-in-Wachs-Emulsion, Öl-in-Wasser-Emulsion und Wasser-in-Öl-Emulsion vorliegt.

46. Verfahren nach einem der nach einem der Ansprüche 26 bis 45, **dadurch gekennzeichnet, dass** die Zusammensetzung ferner mindestens ein flüchtiges Öl enthält.

47. Verfahren nach einem der nach einem der Ansprüche 26 bis 46, **dadurch gekennzeichnet, dass** die Zusammensetzung ferner mindestens einen emulgierenden grenzflächenaktiven Stoff enthält.

48. Verfahren nach Anspruch 47, **dadurch gekennzeichnet, dass** der emulgierende grenzflächenaktive Stoff in einer Menge von 2 bis 30 Gew.-%, bezogen auf das Gesamtgewicht der Zusammensetzung, enthalten ist.

49. Verfahren nach einem der nach einem der Ansprüche 26 bis 48, **dadurch gekennzeichnet, dass** die Zusammensetzung ferner mindestens einen Zusatzstoff enthält, der unter den Vitaminen, Spurenelementen, beruhigenden Stoffen, Maskierungsmitteln, Parfums, Ölen, Vitaminen, Verdickungsmitteln, Proteinen, Ceramiden, Weichmachern, Kohäsionsmitteln, Alkalisierungs- und Ansäuerungsmitteln, Füllstoffen, Pigmenten, Emollientien, Konservierungsmitteln oder deren Gemischen ausgewählt ist.

50. Zusammensetzung nach einem der Ansprüche 26 bis 49, **dadurch gekennzeichnet, dass** die Zusammensetzung eine Zusarnmensetzung zum Schminken oder eine kosmetische Zusammensetzung zur Pflege von Keratinsubstanzen ist.

51. Verwendung von Fasern und eines filmbildendes Polymers in Form von Partikeln in wässriger Dispersion in einer kosmetischen Zusammensetzung zum Schminken oder zur Pflege von Keratinsubstanzen, um auf den Keratinsubstanzen einen Film zu bilden, der insbesondere unter Reibung gegenüber Wasser und/oder gegenüber Tränen und/oder gegenüber Schweiß beständig ist.

## Claims

1. Cosmetic makeup or care process for keratin materials, comprising the application to the keratin materials of a composition comprising, in a physiologically acceptable medium, a film-forming polymer in the form of particles in aqueous dispersion and fibres, **characterized in that** the film-forming polymer is a polyurethane.

2. Process according to Claim 1, **characterized in that** the polyurethane is chosen from the group formed by polyester-polyurethanes and polyether-polyurethanes.

3. Process according to either of Claims 1 and 2, **characterized in that** the polyurethane is an anionic polyurethane.

4. Process according to any one of the preceding claims, **characterized in that** the polyurethane is capable of forming a film which has a hardness ranging from 10 seconds to 200 seconds.

5. Process according to any one of the preceding claims, **characterized in that** the polyurethane is present in a content, by weight of solids, ranging from 1% to 60% by weight and better still from 2% to 25% by weight, relative to the total weight of the composition.

6. Process according to any one of the preceding claims, **characterized in that** the fibres are chosen from silk, cotton, wool or flax fibres, cellulose fibres, polyamide fibres, viscose fibres, acetate fibres, in particular rayon acetate fibres, poly(p-phenyleneterephthalamide) fibres, acrylic fibres, in particular polymethyl methacrylate or poly(2-hydroxyethyl methacrylate) fibres, polyolefin fibres and in particular polyethylene or polypropylene fibres, silica fibres, carbon fibres, in particular in graphite form, polytetrafluoroethylene fibres, insoluble collagen fibres, polyester fibres, polyvinyl chloride or polyvinylidene chloride fibres, polyvinyl alcohol fibres, polyacrylonitrile fibres, chitosan fibres, polyurethane fibres, polyethylene phthalate fibres, or fibres formed from blends of polymers.

7. Process according to any one of the preceding claims, **characterized in that** the fibres are fibres of synthetic origin.

8. Process according to any one of the preceding claims, **characterized in that** the fibres are polyamide or cellulose fibres.

9. Process according to any one of the preceding claims, **characterized in that** the fibres have a length ranging from 0.1 mm to 10 mm and preferably from 1 mm to 5 mm.

10. Process according to any one of the preceding claims, **characterized in that** the fibres have a cross section which is within a circle of diameter ranging from 500 nm to 500 µm.

11. Process according to any one of the preceding claims, **characterized in that** the fibres have a circular or polygonal cross section.

12. Process according to any one of the preceding claims, **characterized in that** the fibres are present in a content ranging from 0.1% to 10% by weight and better still from 0.3% to 5% by weight relative to the total weight of the composition.

13. Process according to any one of the preceding claims, **characterized in that** the composition also contains a wax.

14. Process according to Claim 13, **characterized in that** the wax is present in a content ranging from 0.5% to 40% by weight, preferably from 5% to 30% by weight and better still from 10% to 25% by weight relative to the total weight of the composition.

15. Process according to either of Claims 13 and 14, **characterized in that** the wax comprises at least one wax (I) having a melting point ranging from 70°C to 110°C.

16. Process according to Claim 15, **characterized in that** the wax (I) is present in a content ranging from 0.1% to 20% by weight, relative to the total weight of the composition.

17. Process according to any one of the preceding claims 13 to 16, **characterized in that** the wax comprises at least one wax (II) having a melting point of greater than or equal to 45°C and less than 70°C.

18. Process according to Claim 17, **characterized in that** the wax (II) is present in a wax (I)/wax (II) weight ratio ranging from 0.2 to 1.

19. Process according to any one of the preceding claims, **characterized in that** the composition also comprises at least one auxiliary film-forming polymer other than the polyurethane polymer.

20. Process according to any one of the preceding claims, **characterized in that** the composition comprises water in a content ranging from 1% to 95% by weight and better still from 10% to 80% by weight relative to the total weight of the composition.

21. Process according to any one of the preceding claims, **characterized in that** the composition is in the form of a wax-in-water, water-in-wax, oil-in-water or water-in-oil emulsion.

22. Process according to any one of the preceding claims, **characterized in that** the composition also comprises at least one volatile oil.

23. Process according to any one of the preceding claims, **characterized in that** the composition also comprises at least one emulsifying surfactant.

24. Process according to Claim 23, **characterized in that** the emulsifying surfactant is present in a content ranging from 2% to 30% by weight, relative to the total weight of the composition.

25. Process according to any one of the preceding claims, **characterized in that** the composition comprises at least one additive chosen from the group formed by vitamins, trace elements, softeners, sequestering agents, fragrances, oils, vitamins, thickeners, proteins, ceramides, plasticizers, cohesion agents, acidifying or basifying agents, fillers, pigments, emollients and preserving agents, and mixtures thereof.

26. Cosmetic composition comprising, in a physiologically acceptable medium, a film-forming polymer in the form of particles in aqueous dispersion, a wax and fibres, **characterized in that** the film-forming polymer is a polyurethane.

27. Composition according to Claim 26, **characterized in that** the polyurethane is chosen from the group formed by polyester-polyurethanes and polyether-polyurethanes.

28. Composition according to Claim 26 or 27, **characterized in that** the polyurethane is an anionic polyurethane.

29. Composition according to any one of Claims 26 to 28, **characterized in that** the polyurethane is capable of forming a film which has a hardness ranging from 10 seconds to 200 seconds.

30. Composition according to any one of Claims 26 to 29, **characterized in that** the polyurethane is present in a content, by weight of solids, ranging from 1% to 60% by weight and better still from 2% to 25% by weight, relative to the total weight of the composition.

31. Composition according to any one of Claims 26 to 30, **characterized in that** the fibres are chosen from silk, cotton, wool or flax fibres, cellulose fibres, polyamide fibres, viscose fibres, acetate fibres, in particular rayon acetate fibres, poly(p-phenyleneterephthalamide) fibres, acrylic fibres, in particular polymethyl methacrylate or poly(2-hydroxyethyl methacrylate) fibres, polyolefin fibres and in particular polyethylene or polypropylene fibres, silica fibres, carbon fibres, in particular in graphite form, polytetrafluoroethylene fibres, insoluble collagen fibres, polyester fibres, polyvinyl chloride or polyvinylidene chloride fibres, polyvinyl alcohol fibres, polyacrylonitrile fibres, chitosan fibres, polyurethane fibres, polyethylene phthalate fibres, or fibres formed from blends of polymers.

32. Composition according to any one of Claims 26 to 31, **characterized in that** the fibres are fibres of synthetic origin.

33. Composition according to any one of Claims 26 to 32, **characterized in that** the fibres are polyamide or cellulose fibres.

34. Composition according to any one of Claims 26 to 33, **characterized in that** the fibres have a length ranging from 0.1 mm to 10 mm, preferably from 1 mm to 5 mm.

35. Composition according to any one of Claims 26 to 34, **characterized in that** the fibres have a cross section which is within a circle of diameter ranging from 500 nm to 500 µm.

36. Composition according to any one of Claims 26 to 35, **characterized in that** the fibres have a circular or polygonal cross section.

37. Composition according to any one of Claims 26 to 36, **characterized in that** the fibres are present in a content ranging from 0.1% to 10% by weight and better still from 0.3% to 5% by weight relative to the total weight of the composition.

38. Composition according to any one of Claims 26 to 37, **characterized in that** the wax is present in a content ranging from 0.5% to 40% by weight, preferably from 5% to 30% by weight and better still from 10% to 25% by weight relative to the total weight of the composition.

39. Composition according to any one of Claims 26 to 38, **characterized in that** the wax comprises at least one wax (I) having a melting point ranging from 70°C to 110°C.

40. Composition according to Claim 39, **characterized in that** the wax (I) is present in a content ranging from 0.1% to 20% by weight, relative to the total weight of the composition.

41. Composition according to any one of the preceding Claims 26 to 40, **characterized in that** the wax comprises at least one wax (II) having a melting point of greater than or equal to 45°C and less than 70°C.

42. Composition according to Claim 41, **characterized in that** the wax (II) is present in a wax (I)/wax (II) weight ratio ranging from 0.2 to 1.

43. Composition according to any one of Claims 26 to 42, **characterized in that** the composition also comprises at least one auxiliary film-forming polymer other than the polyurethane polymer.

44. Composition according to any one of Claims 26 to 43, **characterized in that** the composition comprises water in a content ranging from 1% to 95% by weight and better still from 10% to 80% by weight relative to the total weight of the composition.

45. Composition according to any one of Claims 26 to 44, **characterized in that** the composition is in the form of a wax-in-water, water-in-wax, oil-in-water or water-in-oil emulsion.

46. Composition according to any one of Claims 26 to 45, **characterized in that** the composition also comprises at least one volatile oil.

47. Composition according to any one of Claims 26 to 46, **characterized in that** the composition also comprises at least one emulsifying surfactant.

48. Composition according to Claim 47, **characterized in that** the emulsifying surfactant is present in a content ranging from 2% to 30% by weight, relative to the total weight of the composition.

49. Composition according to any one of Claims 26 to 48, **characterized in that** the composition comprises at least one additive chosen from the group formed by vitamins, trace elements, softeners, sequestering agents, fragrances, oils, vitamins, thickeners, proteins, ceramides, plasticizers, cohesion agents, acidifying or basifying agents, fillers, pigments, emollients and preserving agents, and mixtures thereof.

50. Composition according to any one of Claims 26 to 49, **characterized in that** the composition is a makeup composition or a cosmetic care composition for keratin materials.

51. Use of a film-forming polyurethane polymer in the form of particles in aqueous dispersion and fibres in a cosmetic makeup or care composition for keratin materials, in order to obtain a film deposited on the keratin materials that is resistant to water, in particular while rubbing, and/or to tears and/or to perspiration.
